# EUROPEAN PATENT SPECIFICATION

(11) **EP 2 886 117 B1**
(45) Date of publication and mention of the grant of the patent: **21.08.2019**
(21) Application number: 14425154.3
(22) Date of filing: 17.12.2014
(51) Int. Cl.: A61K 31/4164, A61K 31/4168, A61K 31/77, A61K 9/06, A61K 9/00, A61K 47/26, A61K 47/10, A61K 47/32, A61K 47/38, A61P 15/12, A61P 31/04

(54) **Topical compositions for the treatment of Gardnerella vaginalis infections**
Topische Zusammensetzungen zur Behandlung von Gardnerella-Vaginalis-Infektionen
Compositions topiques pour le traitement des infections avec Gardnerella vaginalis

(30) Priority: 17.12.2013 IT RM20130690
(43) Date of publication of application: 24.06.2015
(73) Proprietor: Over S.r.l., 53100 Siena (SI) (IT)
(72) Inventor: Figura, Natale, 53100 Siena (SI) (IT)
(74) Representative: Banchetti, Marina

(56) References cited:
- WO-A1-2011/148405
- US-A1- 2005 186 142
- KAMAL DUA: "Application of model Independent approach on in vitro release of extemporaneously prepared semisolid formulations containing Metronidazole with marketed silver sulfadiazine 1% cream, USP: a comparative study", BULLETIN OF PHARMACEUTICAL RESEARCH, vol. 3, no. 1, April 2013 (2013-04), pages 1-5, XP002722137,
- JONES B M ET AL: "In-vitro and in-vivo activity of metronidazole against Gardnerella vaginalis, Bacteroides spp. and Mobiluncus spp. in bacterial vaginosis", JOURNAL OF ANTIMICROBIAL CHEMOTHERAPY, vol. 16, no. 2, 1 August 1985 (1985-08-01) , pages 189-197, XP009177066, OXFORD UNIVERSITY PRESS, GB ISSN: 0305-7453
- AROUTCHEVA A A ET AL: "Antimicrobial protein produced by vaginal Lactobacillus acidophilus that inhibits Gardnerella vaginalis", INFECTIOUS DISEASES IN OBSTETRICS AND GYNECOLOGY, vol. 9, no. 1, 1 January 2001 (2001-01-01) , pages 33-39, XP009177072, WILEY-LISS, NEW YORK, NY, US ISSN: 1064-7449
- Anonymous: "Flagyl(R) (metronidazole) extended release tablets, 750 mg", U.S. Food and Drug Administration , June 2013 (2013-06), pages 1-16, XP002722138, Retrieved from the Internet: URL:http://www.accessdata.fda.gov/drugsatf da_docs/label/2013/020868s009lbl.pdf [retrieved on 2014-03-19]

## Description

### Field of the invention

The present invention concerns topical compositions for treating infections from *Gardnerella vaginalis.* More specifically, the invention concerns the use, in the therapy of bacterial vaginosis, of semi-solid antibiotic compositions or combinations having metronidazole and polysorbate as active ingredients, the former being an antibiotic already in use for the treatment of bacterial vaginosis, and the latter being a known emulsifying agent/non-ionic surfactant, having the function of potentiating the anti-infective activity of metronidazole and being active as anti-relapse agent.

### Background of the invention

As is known, the normal vaginal flora consists of both aerobic and anaerobic bacteria. The predominant microorganisms are those of the genus *Lactobacillus* (facultative anaerobe), representing approximately 95% of all the present bacteria. These organisms provide a defense against infections, maintaining an acid pH in the vagina, with normal values of less than 4.5. The woman lactobacilli in normal conditions tend to contain more phenotypes that produce hydrogen peroxide, which damages the pathogenic micro-organisms, thus preventing vaginal colonization by such organisms.

Bacterial vaginosis (BV) is one of the most frequent diseases of the vaginal area, with a prevalence of around 20% of women of childbearing age. It is a syndrome characterized by depletion of bacterial microorganisms of the normal vaginal flora, primarily lactobacilli, with simultaneous increase, up to 100-1000 times the normal amount, of vaginal pathogenic bacteria, in particular facultative anaerobes, such as *Gardnerella vaginalis,* and obligate anaerobes, as *Mycoplasma hominis, Mobiluncus spp.* etc .. The disease often has a insidious trend: in some women it begins without symptoms, while in most cases only mild disorders occur, such as discharge, itching or local irritation, although it is always accompanied by detectable phenomena that contribute to formulate a reliable diagnosis, including a rise in vaginal pH to values greater than 4.5 and homogeneous vaginal secretions, having a typical "fishy" odor, due to the presence of amines (Hill GB, et al., Bacteriology of the vagina. Scandinavian Journal of Urology and Nephrology 1984; 86(Suppl):23-29).

Despite the fact that its appearance is sometimes underestimated, this disorder can be complicated by pelvic inflammatory disease or endometritis (Ness RB, et al. Bacterial vaginosis and risk of pelvic inflammatory disease. Obstetrical and Gynecological Survey 2005;60:99-1 00).

Bacterial vaginosis can also complicate a pregnancy, resulting in premature rupture of membranes, preterm births and abortions (Leitich H, et al. Bacterial Vaginosis as a risk factor for pre-term delivery: a meta analysis. American Journal Of Obstetrics And Gynecology 2003;189:139-47). It may even interfere with *in vitro* fertilization, reducing the number of egg cells implanted or causing an early pregnancy loss (Eckert LO, et al., Relationship of vaginal bacteria and inflammation with conception an early pregnancy loss following in-vitro fertilization. Infectious Diseases in Obstetrics and Gynecology 2003;11:11-17).

As already noted, the pathogenesis of VB consists of an excessive growth of facultative anaerobe and obligate anaerobe pathogenic bacteria compared to the normal vaginal bacterial flora which, as is known, is mostly formed by lactobacilli. Since the development of lactobacilli is accompanied by lactic acid production with a consequent reduction of the vaginal pH, it is believed that the acid environment and, possibly, the bacterial competition occurring in other ways could prevent colonization by the microorganisms responsible for bacterial vaginosis (Klebanoff SJ, et al., Control of the Microbial flora of the vagina by H202 generating Lactobacilli. Journal of Infectious Diseases 1991;164:94-100).

In spite of the fact that the microbial species involved in VB are multiple, *G. vaginalis* must always be the first suspect, because of its higher frequency. The drug of choice for the treatment of this disorder is, therefore, metronidazole, an antibiotic very active against anaerobe micro-organisms and in particular against *G*. *vaginalis,* which is at the same time inactive against lactobacilli (Marrazzo JM. Evolving issues in understanding and treating bacterial vaginosis. Expert Review of Anti-Infective Therapy 2004;2:913-22; CDC sexually transmitted diseases treatment guidelines 2006. Morbidity And Mortality Weekly Report 2006;55:50-2).

The most common antibiotic treatments for VB consist of oral preparations of metronidazole or clindamycin, to which 80-90% of women show a positive response. In particular, clindamycin shows a better antibacterial activity compared to metronidazole, but the latter has the advantage of being less harmful to lactobacilli. 15-30% of treated patients, however, undergoes recurrence within three months, and in those with recurrent VB the initial response to treatment is further lower.

According to what reported in the literature, in the current treatment of VB metronidazole is administered orally, in particular with a dosage of 500 mg twice a day for seven consecutive days or, according to another protocol (currently not used because of the greater risk of relapse), with a unit dose of 2 g in a single administration (Barbara A. Majeroni, Bacterial Vaginosis: An Update, American Family Physician, 1998, Vol. 57, No. 6). For the anti-infective treatment in the vaginal region metronidazole may also be administered by topical vaginal application: in this case, the dosages listed above were shown to be equivalent to a topical treatment with 0.75% metronidazole vaginal gel, administered daily at doses 5 g for 5 days.

Although metronidazole is a safe drug to use, it can cause several side effects, such as nausea, vomiting, metallic taste, etc .. More rarely, leukopenia and paresthesias have been reported (Trexler MF, et al. Fulminant pseudo-membranous colitis caused by clindamycin phosphate vaginal cream. American Journal of Gastroenterology 1997;92:2112-3).

Notwithstanding the possibility of enhancing the effects of metronidazole by coupling the local with an oral administration, 10-15% of patients are non responsive to initial treatment. Moreover, about two-thirds of women who respond to treatment suffer a relapse (Bradshaw CS, et al. High recurrence rates of bacterial vaginosis over the course of 12 months after oral metronidazole therapy and factors associated with recurrence. Journal of Infectious Diseases 2006;193:1478-86. 15).

The high frequency of relapse and non-healing of a fair proportion of patients have suggested the search for alternative methods of treatment to eradicate *G. vaginalis,* such as the use of probiotics and the use of substances able to increase the sensitivity of this pathogen to metronidazole and of increasing its bioavailability. The use of probiotic lactobacilli, in particular, has been suggested by the observation that these microorganisms are predominant in the healthy vagina, while they are completely absent from the vaginal microbiota in women with bacterial vaginosis (Reid G, et al. Oral probiotics can resolve urogenital infections. FEMS Immunology and Medical Microbiology 2001; 30:49-52). However, yet in 2009 there was not sufficient evidence to conclude that treatment with probiotics is really effective in improving the therapeutic effects of metronidazole (Senok AC, et al., Probiotics for the treatment of bacterial vaginosis, Cochrane Database Syst Rev 2009 Oct 7(4): CD006289).

Kamal Dua (Application of model independent approach on in vitro release of extemporaneously prepared semisolid formulations containing metronidazole with marketed silver sulfadiazine 1% cream, USP: a comparative investigation, Bull. Pharm. Res. 2013, 3(1), 1-5) discloses some experimental topical preparations containing 5 mg of metronidazole for use in the treatment of bacterial vaginitis, one of which includes 5.5 mg of Tween 80 (polysorbate 80) as excipient.

The co-owned international patent publication WO 2011/148405 discloses the use of polysorbate 80 in combination with metronidazole and/or clarithromycin for the treatment of infections from *Helicobacter pylori,* through oral, i.e. systemic, administration.

Despite the availability of therapeutic treatments for vaginal infection supported by *Gardnerella vaginalis,* there is still a considerable need to have available treatments more effective than the current ones, which should be able to solve the cases refractory to treatment with metronidazole. Especially, such treatments should reduce the incidence of relapse after the first antibiotic treatment.

### Summary of the invention

In the frame of the studies that led to the present invention, it has been taken into consideration the possibility of associating metronidazole, administered in topical preparations, with a specific surfactant/emulsifier with potential antibacterial properties, polysorbate 80, which had been tested with positive results as an adjuvant for the oral treatment of eradication of *Helicobacter pylori in* chronic gastritis by the same Applicant (Figura N., et al., Polysorbate 80 and Helicobacter pylori: a microbiological and ultrastructural study. BMC Microbiol 201212:217-228). As is known, the non-ionic surfactants / emulsifiers known with the common name of "polysorbates" and under the trade name Tween are esters of polyoxyethylene (20) sorbitan and lauric, palmitic, stearic or oleic acid, where the number 20 indicates the total number of oxyethylene units in the molecule. In particular, polysorbate 80 or Tween 80 is polyoxyethylene (20) sorbitan monooleate.

Notwithstanding the hints of the prior art to the ability of polysorbate 80 (P80) to enhance the effectiveness of metronidazole against *Helicobacter pylori* in an oral systemic treatment, the mechanism by which such action is exerted on *H. pylori* is closely linked to the biological characteristics of this microorganism: *H. pylori* is the only bacterium resident in the gastric environment, where it resists a pH that varies between about 1 and 4, and lives immobilized on epithelial cells, generally in a biofilm built by it, and colonizes the gastric mucosa. It is a Gram-negative bacterium, which is actually very sensitive to P80. As pointed out in the literature cited above, the synergism shown by the combination P80/metronidazole is based on the damage created in the outer membrane of *H. pylori,* which facilitates the entry of the drug in the periplasmic space, and possibly the inactivation the effusion pumps (Figura et al., *loc. cit*).

On the other hand, *Gardnerella vaginalis* is a Gram-variable bacterium, considered by many to be a Gram positive (therefore without an outer membrane) facultative anaerobic bacterium, living as saprophyte or commensal in the bacterial flora in approximately 30% of normal women, is installed on epithelial cells also wrapped in a biofilm (giving rise to the so-called "clue cells", vaginal epithelial cells coated with bacteria), but does not colonize the vaginal mucus. Effusion pumps have not been described in GV. As already noted, it is believed that the gardnerella infection develops in response to an imbalance in the vaginal ecosystem, in which these microorganisms take priority over the normal vaginal bacterial flora consisting of lactobacilli, which in normal conditions keeps the environment at a pH of 3.8-4.2. Furthermore, it is noteworthy that *G*. *vaginalis* has a sensitivity to polysorbate which is about 1000 times less than that of *Helicobater pylori.*

According to the present invention, it was experimentally demonstrated (as it will be explained in more detail here below) that polysorbate 80, topically intravaginally administered, exerts a marked dissolving activity on the mucin, which is the fundamental component of the mucus of all organs, and/or in respect of mucus phospholipids. It was also found that such action exerted on the vaginal mucus not only improves the diffusion of metronidazole, increasing its concentration at the site of action and the bioavailability, but also allows to obtain unexpectedly good results in terms of effectiveness. In fact, the antibacterial activity exerted on *G. vaginalis* by the combination of polysorbate 80 and metronidazole, within certain limits of quantitative relations between the two agents, is significantly greater than the sum of the effects obtained by using individually each of the two agents, thereby suggesting a synergistic activity of the two agents against this microorganism.

### Detailed description of the invention

Thus, the present invention specifically provides a pharmaceutical composition for the topical treatment of bacterial vaginosis containing, as active ingredients, polysorbate and metronidazole, wherein weight ratio of the polysorbate and metronidazole is 1.2:1 and 5:1. As evidenced by the literature presented, the bacterial vaginoses treated according to this invention are vaginal infections mainly by Gardnerella vaginalis, although the presence of minor populations of other vaginal pathogens, such as various species of *Mobiluncus,* is not excluded.

Preferably, as noted, the polysorbate used is polysorbate 80, also known under the trade name Tween 80, which corresponds chemically to polyoxymethylene (20) sorbitan monooleate. Alternatively, other polysorbates may be used in the proposed formulation, such as in particular polysorbate 60, polysorbate 40 and polysorbate 20, which also demonstrated, albeit to a lesser extent, a synergistic action with metronidazole when administered in relative proportions according to the invention.

The pharmaceutical composition based on polysorbate and metronidazole proposed according to the invention can be formulated into a preparation for topical vaginal application, in particular in the form of a cream, a gel, a foam, pessaries, vaginal tablets, an emulsion or an ointment. The proposed topical formulations of P80 and metronidazole may also be arranged so as to allow constant and prolonged release in time of the two active ingredients.

In particular, a topical intravaginal medication according to the invention may contain, in addition to common excipients, one or more bioadhesive or mucoadhesive vehicles, so as to obtain an increase of the residence time *in situ* of the active ingredients. The bioadhesive or mucoadhesive vehicle can be an agent selected from the group consisting of hydroxypropylcellulose, carbomers, alginates, pectin, xyloglucanes, chitosan, xanthan gum and polycarbophil.

According to some preferred embodiments of the invention, the proposed topical pharmaceutical preparation may contain from 1.4 to 14.0% by weight of polysorbate 80 and from 0.5 to 6% by weight of metronidazole, with the proviso that the weight ratios between polysorbate and metronidazole are between 1.2:1 and 5:1, or preferably, between 1.2:1 and 4:1.

In further preferred manner, the weight ratios between polysorbate and metronidazole are included in the range between 2:1 and 3:1, or better between 1.4:1 and 3:1. According to a preferred embodiment, the weight ratio between metronidazole and polysorbate is equal to 2.8: 1, while according to an alternative choice may be at least 1.4: 1.

In addition to the bioadhesive and mucoadhesive vehicles mentioned above, the topical preparation based on metronidazole and polysorbate 80 according to the invention may comprise further optional ingredients, as thickening agents, antioxidants, stabilizers, surfactants, etc . Furthermore, the preparation may contain preservatives or antimicrobial agents, such as methyl-, propyl-, ethyl-paraben, benzoic acid, benzyl alcohol, and other substances conventional in the pharmaceutical technique, such as excipients and vehicles for preparations for topical administration.

According to another aspect of the present invention, the antibacterial activity of P80 and metronidazole can be applied also by the combined action of two distinct intravaginal topical preparations, administered so as to obtain the predetermined weight ratios between the two active ingredients.

Thus, the present invention further provides a combination of two topical intravaginal preparations containing respectively, as active ingredients, polysorbate 80 and metronidazole for the treatment of *Gardnerella vaginalis,* wherein said two preparations are dosed in ratios by weight of polysorbate 80 to metronidazole between 1.2:1 and 5:1 in each application.

Each of the two preparations can be independently in the form of a cream, a gel, a foam, pessaries, vaginal tablets, an emulsion or an ointment, and preferably, in order to obtain the correct quantitative ratios of the two products in the administration, these preparations are conveniently presented in distinct dosage units, as pessaries or vaginal tablets, or are dispensable in metered amounts of product from a multidose container, as in the case of creams or gels.

In this case, the two formulations of polysorbate 80 and metronidazole will preferably be produced in such a way that said distinct dosage units and said calibrated amounts of product each respectively contain an amount of polysorbate 80 and an amount of metronidazole in a weight ratio from 1.2:1 to 4:1.

The specific features of the invention, as well as the advantageous aspects thereof, will become more apparent with reference to the detailed description presented merely by way of example in the following, together with the results of experiments carried out on it.

### Examples

The following working examples, which show possible formulations based on polysorbate 80 and metronidazole for topical intravaginal treatment of the infection by *Gardnerella vaginalis* as proposed according to the invention, include the formulations relating to two different pharmaceutical presentations:
- Gel product containing metronidazole and polysorbate 80 in a single dosage form;
- Gel product based on polysorbate 80 only, for contemporaneously use to topical or systemic treatment based on metronidazole.

Possible regimens are summarized as follows:
a) metronidazole *per os* twice a day for 5/7 days, associated with pessaries/ gel / foam of polysorbate 80 and metronidazole in the evening for 7 days; preferably administer lactobacilli at the end of therapy;
b) metronidazole *per os* high-dose (2 g) in a single day, associated with pessaries/gel/foam of polysorbate 80 and metronidazole in the evening for 7 days; preferably administer lactobacilli at the end of therapy;
c) pessaries of metronidazole, associated with gel/foam/cream of polysorbate 80, in the evening for 8-10 days; preferably administer lactobacilli at the end of therapy.

Alternatively, a therapeutic method according to the teachings of the present invention can also be implemented with a simultaneous treatment with metronidazole systemically according to known therapeutic patterns, flanked by a topical treatment with polysorbate 80 in the form of a gel, a foam or a cream, in which the proportions between the two active ingredients are administered within the proposed limits according to the invention, that is, it has a weight ratio of the polysorbate and metronidazole between 1.2: 1 and 5: 1.

### Example 1 - Gel preparation, with polysorbate/metronidazole ratio: 1,4:1 (700 mg/500 mg)

The preparations based on P80 and metronidazole whose compositions are shown in the following (where the percentages are by weight) were produced according to generally accepted procedures in the pharmaceutical art. In particular, in the composition indicated in the following, Carbomer is a crosslinked homopolymer of acrylic acid which acts as a gelling agent, Methocel E is a product based on hydroxypropylmethylcellulose used with thickening functions and Phenonip (phenoxyethanol-methyl p-hydroxybenzoate) is a preservative.

The P80 preferably used in the formulations herein is high purity polysorbate 80 for specific use in the pharmaceutical industry, Polysorbate 80 (HX2) (NOF Corporation, JP).

| **Ingredients** | **Weight (%)** |
|---|---|
| Carbomer | 0.95 |
| Methocel E466 (average viscosity) | 0.04 |
| Triethanolamine 99% | q.s. to about pH 4 |
| Metronidazole | 2.50 |
| Polisorbate 80 | 3.50 |
| Propylene glycol | 2.50 |
| Glycerol | 5.00 |
| Phenonip (phenoxyethanol-methyl p-hydroxybenzoate) | 0.12 |
| Purified water | q.s. to 100.00 |

### Example 2 - Gel preparation, with polysorbate/metronidazole ratio: 1.4:1 (700 mg/500 mg)

| **Ingredients** | **Weight (%)** |
|---|---|
| Polycarbophilic acid | 2.00 |
| Glycerol | 30.00 |
| Carbopol 971 p (crosslinked acryilic polymer) | 1.00 |
| Polycarbophilic acid | 0.676 |
| Triethanolamine 99% | q.s. to pH 4-4.5 |
| Polysorbate 80 | 7.00 |
| Metronidazole | 5.00 |
| Purified water | q.s. to 100.00 |

### Example 3 - Gel preparation with polysorbate/metronidazole ratio: 1.4:1 (700 mg/500 mg)

| **Ingredients** | **Weight (%)** |
|---|---|
| Polycarbophilic acid | 2.00 |
| Glycerol | 30.00 |
| Carbopol 974 (carbomer) | 1.00 |
| polycarbophilic acid | 0.676 |
| Triethanolamine 99% | q.s. to pH 4-4,5 |
| Polysorbate 80 | 7.00 |
| Metronidazole | 5.00 |
| Purified water | q.s. to 100.00 |

### Example 4 - Gel preparation, with polysorbate/metronidazole ratio: 2.8:1 (700 mg/250mg)

| **Ingredients** | **Weight (%)** |
|---|---|
| Carbomer | 0.95 |
| Methocel (E466 average viscosity) | 0.04 |
| Triethanolamine 99% | q.s. to about pH 4 |
| Polysorbate 80 | 7.00 |
| Metronidazole | 2.50 |
| Propylene glycol | 2.50 |
| Glycerol | 5.00 |
| Phenonip (phenoxyethanol-methyl p-oxybenzoate) | 0.12 |
| Purified water | q.s. to 100.00 |

### Example 5 - Gel preparation, with polysorbate/metronidazole ratio: 2.8:1 (700 mg/250 mg)

| **Ingredients** | **Weight (%)** |
|---|---|
| Polycarbophilic acid | 2.00 |
| Glycerol | 30.0 |
| Carbopol 971p (crosslinked acryilic polymer) | 1.00 |
| Polycarbophilic acid | 0.676 |
| Triethanolamine 99% | q.s. to pH 4-4,5 |
| Polysorbate 80 | 14.00 |
| Metronidazole | 5.00 |
| Purified water | q.s. to 100.00 |

### Example 6 - Gel preparation, with polysorbate/metronidazole ratio: 2.8:1 (700 mg/250 mg)

| **Ingredients** | **Weight (%)** |
|---|---|
| Polycarbophilic acid | 2.00 |
| Glycerol | 30.00 |
| Carbopol 974 (carbomer) | 1.00 |
| Polycarbophilic acid | 0.676 |
| Triethanolamine 99% | q.s. to pH 4-4,5 |
| Polysorbate 80 | 10,50 |
| Metronidazole | 3.75 |
| Purified water | q.s. to 100.00 |

### Example 7 - Gel preparation, with polysorbate/metronidazole ratio: 4:1 (500 mg/125 mg)

| **Ingredients** | **Weight (%)** |
|---|---|
| Carbomer | 0,95 |
| Methocel (E466 average viscosity) | 0,04 |
| Triethanolamine 99% | q.s. to pH 4 ca. |
| Polysorbate 80 | 5.00 |
| Metronidazole | 1.25 |
| Propylene glycol | 2.50 |
| Glycerol | 5.00 |
| Phenonip (phenoxyethanol-methyl p-oxybenzoate) | 0,12 |
| Purified water | q.s. to 100.00 |

### Example 8 - Gel preparation, with polysorbate/metronidazole ratio: 4:1 (500 mg/125 mg)

| **Ingredients** | **Weight (%)** |
|---|---|
| Polycarbophilic acid | 2.00 |
| Glycerol | 30.00 |
| Carbopol 971 p (crosslinked acryilic polymer) | 1.00 |
| Polycarbophilic acid | 0.676 |
| Triethanolamine 99% | q.s. to pH 4-4,5 |
| Polysorbate 80 | 10.00 |
| Metronidazole | 2.50 |
| Purified water | q.s. to 100.00 |

### Example 9 - Gel preparation, with polysorbate/metronidazole ratio: 4:1 (500 mg/125 mg)

| **Ingredients** | **Weight (%)** |
|---|---|
| Polycarbophilic acid | 2.00 |
| Glycerol | 30.00 |
| Carbopol 974 (carbomer) | 1.00 |
| Polycarbophilic acid | 0.676 |
| Triethanolamine 99% | q.s. to pH 4-4,5 |
| Polysorbate 80 | 8.00 |
| Metronidazole | 2.00 |
| Purified water | q.s. to 100.00 |

In addition to the formulations in which metronidazole and polysorbate 80 are combined in one dosage form, it is also possible to provide formulations containing as active ingredient the polysorbate 80 alone, to be used for example in therapeutic scheme c) mentioned above, or even in combination with systemic therapy based on metronidazole, as mentioned above.

### Example 10 - Polysorbate based gel preparation

Also in this case, the preparations of P80, whose compositions are shown below (wherein the percentages are by weight), were produced according to generally accepted procedures in the pharmaceutical art. Both the P80 and the excipients used in the compositions that follow are the same as the previous examples.

| **Ingredients** | **Weight (%)** |
|---|---|
| Carbomer | 0.95 |
| Methocel (E466 average viscosity) | 0,04 |
| Triethanolamine 99% | q.s. to about pH 4 |
| Polysorbate 80 | 7.00 |
| Propylene glycol | 2.50 |
| Glycerol | 5.00 |
| Phenonip (phenoxyethanol-methyl p-oxybenzoate) | 0.12 |
| Purified water | q.s. to 100.00 |

### Example 11 - Polysorbate based gel preparation

| **Ingredients** | **Weight (%)** |
|---|---|
| Carbomer | 0.95 |
| Methocel (E466 average viscosity) | 0.04 |
| Triethanolamine 99% | q.s. to about pH 4 |
| Polysorbate 80 | 3.50 |
| Propylene glycol | 2.50 |
| Glycerol | 5.00 |
| Phenonip (phenoxyethanol-methyl p-oxybenzoate) | 0.12 |
| Purified water | q.s. to 100.00 |

### Example 12 - Polysorbate based gel preparation

| **Ingredients** | **Weight (%)** |
|---|---|
| Polycarbophilic acid | 2.0 |
| Glycerol | 30.0 |
| Carbopol 971 p (crosslinked acryilic polymer) | 1.0 |
| Polycarbophilic acid | 0.676 |
| Triethanolamine 99% | q.s. to pH 4-4,5 |
| Polysorbate 80 | 14.00 |
| Purified water | q.s. to 100.00 |

### Example 13 - Polysorbate based gel preparation

| **Ingredients** | **Weight (%)** |
|---|---|
| Polycarbophilic acid | 2.0 |
| Glycerol | 30.00 |
| Carbopol 974 (carbomer) | 1.00 |
| Polycarbophilic acid | 0.676 |
| Triethanolamine 99% | q.s. to pH 4-4,5 |
| Polysorbate 80 | 10,50 |
| Purified water | q.s. to 100.00 |

### Assessment of the ability of P80 to dissolve the vaginal mucus

To assess the ability of the polysorbate 80 to dissolve the vaginal mucus , the mucus was put in contact with P80 at different concentrations, using PBS (phosphate buffered saline) as a control.

Samples of 25 µL of undiluted and variously diluted mucus were deposited on a Petri dish of polystyrene, which, immediately after, was inclined by about 60° for 5 seconds; therefore, the distances run by droplets from the starting point were measured (with a caliber). The test was carried out four times for each sample, and gave the results presented in the following table.

**TABLE 1**

| Concentration of P80 in mucus (µg/mL) | Run in mm (average±SD) |
|---|---|
| 10,000 | 35.7±1.4 |
| 1000 | 23.2± 1.7 |
| 500 | 21.2±3.4 |
| 250 | 20.5±1.2 |
| 125 | 16.7±1.5 |
| 62 | 12.7±2.6 |
| 31 | 9.7±2.2 |
| 16 | 6.7±2.0 |
| 8 | 3.5±0.5 |
| Control without polysorbate | 3.2±0.9 |

From the above data it can be seen that even at a concentration of 16 µg / mL of P80, the distance traveled was significantly greater than the control.

### Assessment of the effectiveness of antibacterial associations brought against G. vaginalis

In order to verify the performance of the combinations of active substances proposed according to the invention in the pharmacological treatment of bacterial vaginosis, the species G. vaginalis has been taken into consideration, being the most important etiologic agent in these infections, and the effectiveness of polysorbate 80 has been detected in vitro alone and in combination with metronidazole, comparing it to the effectiveness of metronidazole alone.

A few species of *lactobacilli saccharomyces* have also been taken into account and, in order to evaluate the activity against *G*. *vaginalis* and to test whether their association with metronidazole can increase the potential of antibacterial chemotherapy to this species.

### Materials and methods

### Assay on antibacterial activity against G. vaginalis of P80 and metronidazole, individually and in combination

The two substances were dissolved in BHI-G containing 5% of dimethyl sulfoxide and diluted in BHI-G broth. Then dilutions were made in base 2 in a volume of 100 µL in Microtiter® plates. To evaluate the effect of their association, the "checkerboard" method was used after dilution of one of the two compounds, 100 µL of the second substance were added to each different concentration of the first and then further dilutions were made. Finally, 100 µL of a suspension of *G. vaginalis* containing approximately 2×10⁶ bacteria were added to all wells.

The plates were incubated anaerobically at 37°C for 72 hours. The minimum inhibitory concentration (MIC) was considered as the lowest concentration of the two substances or their association, to which a complete absence of development was observed compared to the negative control, which does not contain any of the two substances.

The type of interaction was quantified by determining the *"Fractional Inhibitory Concentration* FIC)" : (MIC of substance A in combination/MIC of substance A alone) + (MIC of substance B in combination/MIC of substance B alone): a value ≤05 indicating a synergistic effect; > 0.5-1, an additive effect; 1-4 a neutral effect; > 4 an antagonistic effect.

### Antimicrobial activity against G. vaginalis of Lactobacillus casei, Lactobacillus acidophilus, Lactobacillus paracasei subsp. paracasei F19 and Saccharomyces boulardii

The strains of *L. casei, L. acidophilus* , *L. paracasei* subsp. *paracasei* F19 e *Saccharomyces boulardii* have been obtained from commercial preparations, rispectively Enterolactis®, Lactovagin® GenefilusF19® and Codex®. The lyophilized content was reconstituted with BHI broth containing 1% glucose (BHI-G); after rehydration the various bacterial suspensions were plated on BHI agar with 5% horse blood (BHI-S). After overnight incubation in normal air at 37°C, the various strains were stored in BHI with 15% glycerol at -80°C.

The antimicrobial activity of the examined species of lactobacilli and saccaromyces was determined in several ways. According to a **first method,** 48 hours culture-broths in BHI-S were centrifuged and the supernatant was sterilized by filtration with a Millipore filter of 0.22 µ and diluted to double up to 1:64 in PBS pH 7.4, using Microtiter® plates. To each dilution a suspension of *G. vaginalis* was then added, containing about 10⁶ microorganisms (final value). After incubation at 37°C for 72 hours the tubes were inspected for the presence of bacterial growth, i.e. turbidity. The MIC was considered as the lowest dilution at which the total absence of turbidity is noted.

A **second method** to evaluate the possible antibacterial activity of Lactobacilli against *G*. *vaginalis* consisted in seeding a suspension of *G*. *vaginalis* with a buffer plated on BHI-S. Then, on the surface of the agar a 0.2 mL steel sterile cylinder was placed, partially filled with 50 L of a culture-broth of lactobacilli. The plates were then placed to incubate in normal air at 37°C for 48 hours and inspected for the presence of a halo of inhibition of G. vaginalis growth: the presence of a halo of inhibition would likely indicate an antibacterial effect.

Finally, regardless of a probable antibacterial power of culture-broths of lactobacilli and saccharomycetes against gardnerella, the possibility that the culture filtrate could increase the synergistic activity of the association P80/Metronidazole has been examined. After diluting the P80 and metronidazole, to each well containing a different concentration of the two substances an equal volume of the filtrate to the final dilution, corresponding to 25% of its MIC, was added. After the addition of a suspension of *G*. *vaginalis* (106 microorganisms per well) and incubation at 37°C for 72 hours, the MIC of the combination and of the substances used individually was recorded and compared with the MIC obtained without the filtrate culture-broth.

### Results

### Test of antibacterial activity of P80 and metronidazole, individually and in combination

The MIC of P80 and metronidazole, tested in isolation, against *G*. *vaginalis* was respectively 25 mg/mL and 4 (µg/mL. The MIC of the P80/metronidazole combination was 0,8 mg/0,06 µg per mL.

FIC index was 0,037 indicative of the fact that the association is synergistic.

### Antimicrobial activity against G. vaginalis of L. casei. L. acidophilus, L. paracasei subsp. paracasei F19 and Saccharomyces boulardii

The filtrates of culture-broth of strains of *Lactobacillus* were pursuing an activity for inhibiting the growth of *G*. *vaginalis* at a dilution of 1: 2. At a dilution of 1:4, a slight turbidity of the broth has been observed, while at a dilution of 1:8, the filtrates did not exercise any antimicrobial power.

The filtrates of culture-broth of *S*. *boulardii* exerted only a moderate inhibitory or bactericidal effect against *G*. *vaginalis* (reduction of the bacterial load of about 3 logarithms compared to the control without filtered). The agar diffusion test showed no inhibitory activity towards the gardnerella.

The filtered culture-broth did not increase the antibacterial activity of metronidazole, or P80, or P80/Metronidazole combination, against *G*. *vaginalis.*

### Conclusions

In conclusion:
a) the polysorbate 80 showed a good antibacterial activity towards *G*. *vaginalis* and the ability to increase the antibiotic activity of metronidazole in respect of this species;
b) the combination of polysorbate 80 and metronidazole showed a synergistic effect with a FIC index indicative of a strong antibacterial synergy.

These results suggest that it may be advantageous to employ the metronidazole in the treatment of bacterial vaginosis in association with the polysorbate, for example in the form of pessaries or creams / gel / vaginal foams, with or without lactobacilli. The polysorbate 80 also provides the fatty acids necessary for the growth of lactobacilli, preventing the possibility of recurrence.

In light of the results, you might also consider the possibility of reducing the dosage of metronidazole, given the strong synergism of the combination, avoiding or reducing the side effects of antibiotic treatment.

Lactobacilli showed a modest antibacterial activity *in vitro* against *G*. *vaginalis.* They, however, could help to speed up the bacteriological healing of the infection favoring the return of the vaginal pH to acidity, and helping to avoid superinfection by *Candida vaginalis* (mycosis) and preventing the possibility of recurrences, which are frequent in this type infections.

The present invention has been disclosed with particular reference to some specific embodiments thereof, but it should be understood that modifications and changes may be made by the persons skilled in the art without departing from the scope of the invention as defined in the appended claims.

## Claims

1. A pharmaceutical composition for use in the topical treatment of bacterial vaginosis containing, as active ingredients, polysorbate and metronidazole, wherein the weight ratio of polysorbate to metronidazole is from 1.2:1 to 5:1, and wherein said polysorbate is selected from the group consisting of polysorbate 80, polysorbate 60, polysorbate 40 and polysorbate 20.

2. The topical pharmaceutical composition for the use of claim 1, wherein said bacterial vaginosis is predominantly mostly sustained by *Gardnerella vaginalis.*

3. The topical pharmaceutical composition for the use of claims 1 or 2, wherein said polysorbate is polysorbate 80.

4. The topical pharmaceutical composition for the use claim 3, formulated into a preparation for topical vaginal application in form of a cream, a gel, a foam, pessaries, vaginal tablets, an emulsion or an ointment.

5. The topical pharmaceutical composition for the use claim 4, containing from 1.4 to 14.0% by weight of polysorbate 80 and from 0.5 to 6% by weight of metronidazole, with the proviso that the weight ratios of polysorbate to metronidazole are comprised between 1.2: 1 and 5:1.

6. The topical pharmaceutical composition for the use of claim 5, wherein said weight ratio of polysorbate to metronidazole is comprised between 1.4:1 and 3:1.

7. The combination of two topical intravaginal preparations respectively containing, as active ingredient, polysorbate 80 and metronidazole, for use in the treatment of infections of *Gardnerella vaginalis,* wherein said two preparations are dosed in ratios by weight of polysorbate to metronidazole comprised between 1.2: 1 and 5:1 in each application.

8. The combination of topical preparations for the use according to claim 7, wherein each of said two preparations is independently in the form of a cream, a gel, a foam, pessaries, vaginal tablets, an emulsion or an ointment.

9. The combination of topical preparations for the use according to claim 8, wherein said preparations are presented in single dosage units, as pessaries or vaginal tablets, or are dispensable in metered amounts of product from a multidose container.

10. The combination of topical preparations for the use according to claim 9, wherein said dosage unit and said metered amount of product each contain, respectively, an amount of polysorbate 80 and an amount of metronidazole which are in ratios by weight between 1.2:1 and 5:1 to each other.

11. The combination of topical preparations for the use according to claim 10, wherein said ratio by weight of polysorbate to metronidazole is between 1.4:1 and 3:1.

## Patentansprüche

1. Pharmazeutische Zusammensetzung zur Verwendung bei der topischen Behandlung von bakterieller Vaginose, enthaltend als Wirkstoffe Polysorbat und Metronidazol, wobei das Gewichtsverhältnis von Polysorbat zu Metronidazol 1,2:1 bis 5:1 beträgt und wobei das Polysorbat ausgewählt ist aus der Gruppe, bestehend aus Polysorbat 80, Polysorbat 60, Polysorbat 40 und Polysorbat 20.

2. Topische pharmazeutische Zusammensetzung zur Verwendung nach Anspruch 1, wobei die bakterielle Vaginose überwiegend hauptsächlich durch *Gardnerella vaginalis* aufrechterhalten wird.

3. Topische pharmazeutische Zusammensetzung zur Verwendung nach Ansprüchen 1 oder 2, wobei das Polysorbat Polysorbat 80 ist.

4. Topische pharmazeutische Zusammensetzung zur Verwendung nach Anspruch 3, formuliert zu einem Präparat zur topischen vaginalen Anwendung in Form einer Creme, eines Gels, eines Schaums, von Pessaren, Vaginaltabletten, einer Emulsion oder einer Salbe.

5. Topische pharmazeutische Zusammensetzung zur Verwendung nach Anspruch 4, enthaltend von 1,4 bis 14,0 Gew.-% Polysorbat 80 und von 0,5 bis 6 Gew.-% Metronidazol, mit der Maßgabe, dass die Gewichtsverhältnisse von Polysorbat zu Metronidazol zwischen 1,2:1 und 5:1 liegen.

6. Topische pharmazeutische Zusammensetzung zur Verwendung nach Anspruch 5, wobei das Gewichtsverhältnis von Polysorbat zu Metronidazol zwischen 1,4:1 und 3:1 liegt.

7. Kombination aus zwei topischen intravaginalen Präparaten, die als Wirkstoff jeweils Polysorbat 80 und Metronidazol enthalten, zur Verwendung bei der Behandlung von Infektionen mit *Gardnerella vaginalis,* wobei die zwei Präparate in Gewichtsverhältnissen von Polysorbat zu Metronidazol dosiert sind, die zwischen 1,2: 1 und 5:1 in jeder Anwendung liegen.

8. Kombination aus topischen Präparaten zur Verwendung nach Anspruch 7, wobei jedes der zwei Präparate unabhängig in Form einer Creme, eines Gels, eines Schaums, von Pessaren, Vaginaltabletten, einer Emulsion oder einer Salbe vorliegt.

9. Kombination aus topischen Präparaten zur Verwendung nach Anspruch 8, wobei die Präparate in Einzeldosierungseinheiten, als Pessare oder Vaginaltabletten vorliegen oder in abgemessenen Mengen eines Produkts aus einem Mehrfachdosisbehälter abgegeben werden können.

10. Kombination aus topischen Präparaten zur Verwendung nach Anspruch 9, wobei die Dosierungseinheit und die abgemessene Produktmenge jeweils eine Menge Polysorbat 80 und eine Menge Metronidazol enthalten, die in Gewichtsverhältnissen zwischen 1,2:1 und 5:1 zueinander liegen.

11. Kombination aus topischen Präparaten zur Verwendung nach Anspruch 10, wobei das Gewichtsverhältnis von Polysorbat zu Metronidazol zwischen 1,4:1 und 3:1 liegt.

## Revendications

1. Composition pharmaceutique pour utilisation dans le traitement topique de la vaginose bactérienne contenant comme ingrédients actifs un polysorbate et du métronidazole, dans laquelle le rapport pondéral du polysorbate au métronidazole est de 1,2:1 à 5:1 et dans laquelle ledit polysorbate est choisi dans le groupe constitué du polysorbate 80, du polysorbate 60, du polysorbate 40 et du polysorbate 20.

2. Composition pharmaceutique topique pour utilisation selon la revendication 1, dans laquelle ladite vaginose bactérienne est entretenue de manière prédominante principalement par la souche *Gardnerella vaginalis.*

3. Composition pharmaceutique topique pour utilisation selon les revendications 1 ou 2, dans laquelle ledit polysorbate est du polysorbate 80.

4. Composition pharmaceutique topique pour utilisation selon la revendication 3, formulée en une préparation pour application vaginale topique sous la forme d'une crème, d'un gel, d'une mousse, de pessaires, de comprimés gynécologiques, d'une émulsion ou d'une pommade.

5. Composition pharmaceutique topique pour utilisation selon la revendication 4, contenant de 1,4 à 14,0 % en poids de polysorbate 80 et de 0,5 à 6 % en poids de métronidazole, à condition que les rapports pondéraux du polysorbate au métronidazole soient compris entre 1,2:1 et 5:1.

6. Composition pharmaceutique topique pour utilisation selon la revendication 5, dans laquelle ledit rapport pondéral du polysorbate au métronidazole est compris entre 1,4:1 et 3:1.

7. Combinaison de deux préparations intravaginales topiques contenant respectivement comme ingrédients actifs du polysorbate 80 et du métronidazole pour utilisation dans le traitement d'infections de *Gardnerella vaginalis,* dans laquelle lesdites deux préparations sont dosées dans des rapports en poids du polysorbate au métronidazole compris entre 1,2:1 et 5:1 dans chaque application.

8. Combinaison de préparations topiques pour utilisation selon la revendication 7, dans laquelle chacune desdites deux préparations se présente indépendamment sous la forme d'une crème, d'un gel, d'une mousse, de pessaires, de comprimés gynécologiques, d'une émulsion ou d'une pommade.

9. Combinaison de préparations topiques pour utilisation selon la revendication 8, dans laquelle lesdites préparations se présentent sous la forme d'unités de dosage individuelles, telles que des pessaires ou des comprimés gynécologiques, ou peuvent être distribuées en quantités mesurées de produit par un récipient à doses multiples.

10. Combinaison de préparations topiques pour utilisation selon la revendication 9, dans laquelle ladite unité de dosage et ladite quantité mesurée de produit contiennent chacune respectivement une quantité de polysorbate 80 et une quantité de métronidazole qui sont dans des rapports mutuels en poids compris entre 1,2:1 et 5:1.

11. Combinaison de préparations topiques pour utilisation selon la revendication 10, dans laquelle ledit rapport en poids du polysorbate au métronidazole se situe entre 1,4:1 et 3:1.
